Europäisches Patentamt

European Patent Office

Office européen des brevets

(11) Publication number: **0 135 171**
**A2**

(12) **EUROPEAN PATENT APPLICATION**

(21) Application number: **84109983.1**

(22) Date of filing: **22.08.84**

(51) Int. Cl.⁴: **A 61 K 45/02**
**A 61 K 9/10**

(30) Priority: **24.08.83 US 525843**

(43) Date of publication of application:
**27.03.85 Bulletin 85/13**

(84) Designated Contracting States:
**AT BE CH DE FR GB IT LI NL**

(71) Applicant: **F. HOFFMANN-LA ROCHE & CO.**
**Aktiengesellschaft**

**CH-4002 Basel(CH)**

(72) Inventor: **Touitou, Elka**
**54 Kubovy**
**Jerusalem(IL)**

(72) Inventor: **Goldberg, Arthur H.**
**143 Montclair Avenue**
**Montclair N.J.(US)**

(74) Representative: **Lederer, Franz, Dr. et al,**
**Patentanwälte Dr. Lederer Franz Meyer-Roxlau Reiner F.**
**Lucile-Grahn-Strasse 22**
**D-8000 München 80(DE)**

(54) Pharmaceutical composition for interferon application.

(57) There is disclosed pseudomonophase, microemulsion compositions for topical application of interferon to the human or animal skin and mucosa. The compositions comprise a therapeutically effective amount of interferon, 30-70% by volume of a surface active agent having a hydrophilic-lipophilic balance (HLB) of from 12-15 and dual solubility in water/oil; 5-45% of a vegetable oil; and 5-45% water. The compositions are highly skin substantive.

EP 0 135 171 A2

F. Hoffmann-La Roche & Co.
Aktiengesellschaft
Basel / Schweiz

RAN 4602/21

## Pharmaceutical composition for interferon application

There are various reports in the literature describing exprimental treatment of a number of diseases by topical application of interferon. Topical application of human leukocyte interferon in stasis ulcers and deep burns was reported by Ikic, et al., International Journal of Clinical Pharmacology, Therapy and Toxicology, Vol. 19, No. 10, 450-452 (1981). The clinical use of human leukocyte interferon in viral infections is described by Ikic, et al. International Journal of Clinical Pharmacology, Therapy and Toxicology, Vol. 19, No. 11, 498-505 (1981), Zagreb, Yugoslavia. Treatment of labial and genital herpes with human leukocyte interferon was reported by Ikic, et al., Proc. Symposium on Clinical Use of Interferon, pages 195-202. In the reported studies, interferon was incorporated in conventional topical formulations, for example, solutions, ointments, creams, oil suspensions, powders, and the like. Therapeutical effect of human leukocyte interferon incorporated into ointment and cream on condylomata acuminata was reported by Ikic, et al; Proc. Symposium on Clinical Use of Interferon, pages 235-238 (Copyright 1975 by the Yugoslav Academy of Sciences and Arts).

European Patent Application No. 77,063 discloses interferon-containing compositions useful for treating herpes simplex viral infections in human. The compositions comprise human interferon and about 0.1% to about 20% by weight of an antiviral surface active agent and a physiologically acceptable carrier. The compositions are topically administered to the affected area.

Grn/17.7.84

- 2 -                    0135171

It would be desirable to provide pharmaceutical compositions which would effectively afford topical administration of interferon to the skin of mucosa of a patient in need of such treatment.

The invention relates to compositions for topical application of interferon to the skin and mucosa of warm-blooded animals. The present invention provides compositions in which proper balance of the partition coefficient of the interferon between the vehicle and the skin, the solubility of the interferon in the vehicle and the diffusion coefficient of the interferon through the vehicle, are achieved by using optimal hydrophilic-lipophilic compositions in which the drug has a high activity and a relatively lower affinity for the carrier. The compositions constitute a pseudomonophase system in which by controlling the oil and the aqueous phases, the physical and penetrating properties may be varied. This invention provides compositions which comprise surface active agents having a hydrophilic-lipophilic balance and water solubility adequate to hold together the oil and the aqueous phase in a clear, micellar pseudomonophase system within the viscosity range as a function of the phase ratios.

Although the systems of the present invention comprise different proportions of oil, water and surfactant, they are not classic emulsions in which the oil phase is separated from the aqueous phase at the interface by surfactant monolayers.

The compositions of the invention comprise a therapeutically effective amount of interferon for topical administration; 30-70% by volume of a surface active agent possessing a hydrophilic-lipophilic balance of from 12-15 and dual solubility in water and oil, 5-45% by volume of a vegetable oil and 5-45% by volume water. The composition may also contain compatible additives used in topical formulations.

The term interferon as used herein encompasses natural and recombinant interferons, for example leukocyte, fibroblast and immune interferon, as well as analogues and derivatives thereof. A preferred interferon is recombinant leukocyte interferon A.

The surface active agents utilized in the present invention are characterized as possessing a hydrophilic-lipophilic balance (HLB) value of from 12-15. The surface active agent has dual solubility in water/oil, i.e. it is soluble in water and soluble, miscible or dispersible in oil. Surface active agents which are useful in preparing the compositions of this invention are polyethylene glycol derivatives of castor oil composed on average of 25-36 moles of ethylene oxide per mol of castor oil. The preferred surface active agent is polyethylene glycol derivatives of castor oil composed on average of 30 moles of ethylene oxide per mol of castor oil, which is available in the trade under the designation PEG-30 castor oil and Emulphor$^R$ EL-620, a trademark of GAF.

The oil component of the compositions of the present invention are vegetable oils. Suitable oils include castor oil, coconut oil and oils consisting of fractionated triglycerides derived from coconut oil, available in the trade under the designation Neobee Oil M5, available from Kay Fries.

There may also be incorporated into the composition of the present invention additional ingredients or pharmaceutical adjuvants recognized in the art of pharmaceutical compounding of topical formulations. Pharmaceutical adjuvants that may be used include stabilizers, such as human serum albumin; preservatives, such as phenol, methyl paraben, propyl paraben; antioxidants, such as butylated hydroxy anisole, butylated hydroxy toluene; and the like. The choice of such materials and the amounts to be utilized are considered to be within the purview of one skilled in the art. It is to be borne in mind, however, that such conventional pharmaceutical adjuncts which might adversely affect the pseudomonophase balance of the topical compositions of the present invention are not suitable for use herein.

The phase composition of the pseudomonophase compositions of the present invention may vary from 9:9 to 9:5 surfactant to aqueous-oil phases. The phase composition of the aqueous phase to the oil phase may vary from 1:8 to 8:1. A preferred ratio of ingredients is 9:2:4, surfactant to water to oil.

The topical compositions of this invention are prepared by conventional techniques well established in the art. The surfactant and oil ingredients are intimately mixed until homogeneous and no gel agglomeration occurs. The interferon component is dissolved in water and the aqueous solution is added in one portion with stirring to the oil-surfactant component. Formation of the component phases is carried out at room temperature.

The appearance of the compositions of the present invention are viscous liquids having varying degrees of viscosity. The compositions exhibit good substantivity and adherence to the skin and mucosa. The compositions are easily washed from the skin tissues and fabrics.

The manner in which the topical compositions are employed will be readily apparent from the foregoing description, as well as from the examples which follow. The novel compositions will be applied in a thin layer to the subject to be treated from single dose or multiple dose containers. Application of the composition may be accomplished by use of a cotton swab, soft brush, sponge or applied directly from a container such as an expecially designed container, for example a Hill Top Chamber$^{TM}$ available from Hill Top Research Inc., Cincinnati, Ohio. Obviously, the product can be used in either greater or lesser quantities, if so desired.

Skin penetration studies can be carried out with the formulations of the present invention utilizing the horizontal diffusion cell method described by Durrheim et al., J. Pharm. Sci., 69, 781 (1980).

Excised human abdominal skin, obtained from cadavers, was kept frozen (-15°C) until used for the experiments. Three hours before the experiment, the skin was defrosted, the subcutaneous fat was removed to the greatest extent possible and cleaned under running distilled water. Before and at the end of each experiment, the skin was checked visually for integrity of the stratum corneum. Adequate marked areas of skin were sectioned off and clamped between the half diffusion cells with the stratum corneum facing the donor compartment and the dermis facing the receiver. The effective skin diffusion area was 0.57 cm$^2$. The donor compartment was filled with 3 ml of interferon solution and the receiver compartment contained 3 ml of placebo. The solutions on both sides of the cells were stirred with small magnets at 150 rpm. The cells were immersed in a constant temperature bath maintained at 37°C. Samples were withdrawn at 1, 4, 22 and 45 hours.

All 3 ml of the receiver compartment were exchanged at each time with fresh release medium. This exchange ensures skin conditions in the system.

For a fuller understanding of the nature of this invention, reference may be had to the following examples which are given merely as further illustrations of the invention and are not to be construed in a limiting sense.

## Example 1

Three compositions containing $70 \times 10^6$ units/ml recombinant leukocyte interferon-A (rIFL-αA) and 60% by weight of polyethylene glycol derivative of castor oil having an average of 30 moles of ethylene oxide (Emulphor EL-629) and different proportions of oil, fractionated triglycerides derived from coconut oil (Neobee Oil M5) were prepared. The compositions were prepared by intimately mixing the surfactant and oil components until homogeneous at room temperature. The interferon was dissolved in the water at room temperature. The aqueous interferon solution was added in one portion with stirring to the surfactant--oil component. Table I reports the physical state and viscosity of the formulation according to this example

## Table I

Formulations Containing $70 \times 10^6$ units/ml rIFL-αA in a Microemulsion System Hydrophilic-Lipophilic Ratio (Volume)

| Formulation | Aqueous Phase(%) | | Oily Phase(%) | | Surfactant(%) | | Physical State | Viscosity |
|---|---|---|---|---|---|---|---|---|
| I | 1 | 6.6 | 5 | 33.3 | 9 | 60.0 | clear liquid | + |
| II | 2 | 13.3 | 4 | 26.6 | 9 | 60.0 | clear liquid | ++ |
| III | 3 | 20.0 | 3 | 20.0 | 9 | 60.0 | clear viscous liquid | +++ |

<u>Scale:</u>

  + low viscosity

  ++ moderate viscosity

 +++ high viscosity

<u>Example 2</u>

A composition of the present invention was prepared from the following ingredients:

<u>Formulation A:</u>

$^{125}$I-rIFL-αA (2.2x10$^6$ cpm)  200 μl ~ 10,000 units

rIFL-αA solution                         100 μl ~ 10$^9$ u/ml

Emulphor EL-620                          500 μl

Neobee oil M5                            500 μl

Skin penetration studies were carried out using the Franz diffusion cell method described by Franz, J. Invest. Dermatol., <u>64</u>, 190 (1975). This is basically similar to the horizontal cell method with the following differences: the cells are vertical, the receiver volume is 8-10 ml, only the receiver medium is stirred at 600 rpm. by means of a small magnet and the effective surface area is 1.75 cm$^2$.

Interferon was found in the epidermis and dermis of cadaver abdominal skin at the end of a 50 hour penetration experiment in Franz cells. The results presented below show that the highest concentration was found in the epidermis.

Distribution of radiolabelled Interferon (cpm) between skin layers and the receiver compartments of the Franz cell:

| Donor | | | Receiver | | | Skin | |
| Compo sition | Volume | cpm | Compo sition | Volume | Epi cpm | dermis cpm | Dermis cpm |
| --- | --- | --- | --- | --- | --- | --- | --- |
| Formu lation A | 1.3ml | $2.2 \times 10^6$ | Placebo | 9 ml | 5715 | 25918 | 3779 |

Area = 1.75 $cm^2$

The placebo solution used in this example had the following components:

| | |
| --- | --- |
| Sodium chloride | 9.0 mg |
| Phenol | 3.0 mg |
| Human serum albumin | 5.0 mg |
| Distilled water, q.s. | 1.0 ml |

The solution was adjusted to pH 7 with buffer solution.

## Example 3

Compositions containing varying proportions of surfactant (Emulphor EL-260) oil and water were prepared to determine the appearance and consistency of the compositions. The results are set forth in Table 2.

## Table 2: Phase Composition and Appearance
## of Applications

| S:W-O | Emulphor | Aqueous Phase | Oily Phase | Appearance | Consistency |
|---|---|---|---|---|---|
| 9:9 | 9 | 1 | 8 | clear | liquid |
|  | 9 | 2 | 7 | clear | liquid |
|  | 9 | 3 | 6 | clear | gel |
|  | 9 | 8 | 1 | hazy | gel |
| 9:8 | 9 | 2 | 6 | clear | liquid |
| 9:6 | 9 | 1 | 5 | clear | liquid |
|  | 9 | 2 | 4 | clear | liquid |
|  | 9 | 3 | 3 | clear | viscous liquid |
|  | 9 | 4 | 2 | hazy | gel |
| 9:5 | 9 | 3 | 2 | clear | gel |
|  | 9 | 1 | 4 | clear | liquid |
| 6:10 | 6 | 2 | 8 | clear | viscous liquid |
|  | 6 | 3 | 7 | hazy | gel |
| 6:6 | 6 | 1 | 5 | clear | liquid |
|  | 6 | 2 | 4 | clear | gel |
|  | 6 | 3 | 3 | hazy | gel |
| 4:10 | 4 | 1 | 9 | clear | liquid |
|  | 4 | 2 | 8 | hazy | gel – separate |
| 4:6 | 4 | 1 | 5 | clear | gel |
|  | 4 | 2 | 4 | hazy | liquid |
| 4:4 | 4 | 1 | 3 | clear | liquid |

S – Surfactant

W-O – Aqueous – oily phases

## Example 4

Compositions containing varying proportions of vegetable oil (Neobee M5 oil), Emulphor EL 620 and water were prepared as described in Example 1 with the exception that the interferon ingredient was omitted. The following table sets forth the appearance of the resulting composition:

## TABLE

Appearance of Preparations (placebo)
Containing Different Proportions
of Vegetable Oil (Neobee M5 Oil), Emulphor EL 620 and Water

| No. | Emulphor EL 620 % v/v | Neeobee Oil M5 % v/v | $H_2O$ % v/v | Other additives % v/v | Appearance of the final preparation |
|-----|-----|-----|-----|-----|-----|
| 1 | 20 | 10 | 70 | | Hazy |
| 2 | 20 | 70 | 10 | | Two phases |
| 3 to 11 | 20 | 5 | 75 | | Low viscosity, slightly hazy |
| 4 | 20 | 75 | 5 | | Two phases |
| 5 | 20 | 40 | 40 | | Emulsions white |
| 6 | 20 | 40 | 20 | 20 Propylene glycol | Nonstable emulsion |
| 7 | 60 | 27 | 13 | | Clear, viscous, yellowish |
| 8 | 18 | 10 | 72 | | White, hazy |
| 9 | 18 | 75 | 7 | | Two phases |
| 10 | 30 | 60 | 10 | | Two phases |
| 11 | 30 | 10 | 60 | | Clear, yellowish |
| 12 | 30 | 5 | 65 | | Clear, yellowish |
| 13 | 30 | 65 | 5 | | Two phases |
| 14 | 30 | 50 | 20 | | Two phases |

## Example 5

A composition was prepared containing 60% by volume of
Emulphor EL-620, 27% by volume of parafin oil and 13% by
volume of water. The resulting composition did not form a
pseudomono phase system but rather separates into two
phases.

## Claims

1. A pharmaceutical composition for administration of interferon into or through the skin of a warm-blooded animal comprising a therapeutically effective amount of interferon, 30% to 70% by volume of a surface active agent having a hydrophilic-lipophilic balance (HLB) value of from 12-15 and dual solubility in water/oil, 5-45% by volume of a vegetable oil and 5-45% by volume water.

2. The composition of claim 1 wherein the interferon is a natural or recombinant leukocyte or fibroblast interferon.

3. The composition of claim 2 wherein the interferon is recombinant leukocyte interferon A.

4. The composition of claim 1 wherein the surface active agent is a polyethylene glycol derivative of castor oil composed on average of 30 moles of ethylene oxide per mol of castor oil.

5. The composition of claim 1 wherein the vegetable oil is an oil consisting of fractional triglyceride derived from coconut oil.

6. The composition of claim 1 wherein the composition also contains pharmaceutical adjuvants.

7. The composition of claim 6 wherein the pharmaceutical adjuvant is a preservative selected from the group consisting of phenol, methyl paraben and propyl paraben.

8. The composition of claim 7 wherein the preservative is phenol.

9. The composition of claim 6 wherein the pharmaceutical adjuvant is human serum albumin.

\*\*\*